# EUROPÄISCHE PATENTANMELDUNG

(11) **EP 3 649 978 A1**
(43) Veröffentlichungstag der Anmeldung: **13.05.2020**
(21) Anmeldenummer: 18204816.5
(22) Anmeldetag: 07.11.2018
(51) Int. Cl.: A61C 1/00

(54) **VERFAHREN ZUR WEBBASIERTEN DATENÜBERTRAGUNG FÜR EIN DENTALES ODER DENTALCHIRURGISCHES BEHANDLUNGS- ODER DIAGNOSESYSTEM UND DERARTIGES BEHANDLUNGS- ODER DIAGNOSESYSTEM**

(71) Anmelder: W & H Dentalwerk Bürmoos GmbH, 5111 Bürmoos (AT)
(72) Erfinder: SCHÖCHL, Andreas, 5163 Mattsee (AT); REITER, Michael, 5061 Elsbethen (AT)
(74) Vertreter: Benda, Ralf

(57) **Zusammenfassung**

Es ist ein Verfahren zur Übertragung von zumindest einem Datensatz zwischen einem dentalen oder dentalchirurgischen Behandlungs- oder Diagnosegerät (2) und einem externen, außerhalb des dentalen oder dentalchirurgischen Behandlungs- oder Diagnosegeräts (2) angeordneten Speicherelement (5A) beschrieben, bei dem zumindest eine Datensatz webbasiert mittels Internet und eines Cloud-Computing-Netzwerks (5), welches das externe Speicherelement (5A) umfasst, übertragen wird.

Ein entsprechendes dentales oder dentalchirurgisches Behandlungs- oder Diagnosesystem (1) umfasst ein dentales oder dentalchirurgisches Behandlungs- oder Diagnosegerät (2) und eine Sende- und Empfangsvorrichtung (3) zur kommunikativen Verbindung mit einer Fernbedienungs-Mensch-Maschine-Schnittstelle (4). Die Sende- und Empfangsvorrichtung (3) ist ausgebildet, eine webbasierte Anbindung über das Internet und ein Cloud-Computing-Netzwerk (5) an die Fernbedienungs-Mensch-Maschine-Schnittstelle (4) bereitzustellen.

## Beschreibung

Die vorliegende Erfindung betrifft ein Verfahren zur webbasierten Übertragung zumindest eines Datensatzes für ein dentales oder dentalchirurgisches Behandlungs- oder Diagnosesystem und ein derartiges Behandlungs- oder Diagnosesystem.

Aus der Patentanmeldung US 2013/0116805 A1 ist eine dentale Behandlungsvorrichtung bekannt, die über eine tragbare Mensch-Maschine-Schnittstelle, zum Beispiel ein Smartphone, steuerbar ist. Dazu sind auf dem Smartphone die zur Steuerung benötigten Programme und Daten lokal gespeichert. Der Datenaustausch zwischen der dentalen Behandlungsvorrichtung und dem Smartphone erfolgt über eine lokale Kommunikationsverbindung.

Ein Nachteil dieses Behandlungssystems liegt darin, dass die Steuerung der dentalen Behandlungsvorrichtung auf jene Mensch-Maschine-Schnittstelle eingeschränkt ist, auf der die dafür benötigten Programme und Daten gespeichert sind. Ist diese Mensch-Maschine-Schnittstelle nicht verfügbar, so kann die dentale Behandlungsvorrichtung nicht gesteuert oder Daten daraus abgefragt werden.

Der vorliegenden Erfindung liegt daher die Aufgabe zugrunde, ein Verfahren zur Übertragung zumindest eines Datensatzes für ein dentales oder dentalchirurgisches Behandlungs- oder Diagnosesystem und ein entsprechendes dentales oder dentalchirurgisches Behandlungs- oder Diagnosesystem zu schaffen, die einen weniger eingeschränkten Zugang zu dem dentalen oder dentalchirurgischen Behandlungs- oder Diagnosesystem ermöglichen. Insbesondere soll die Kommunikation mit dem dentalen oder dentalchirurgischen Behandlungs- oder Diagnosesystem nicht an eine bestimmte Mensch-Maschine-Schnittstelle gebunden sein.

Diese Aufgabe wird gemäß der vorliegenden Erfindung durch ein Verfahren zur Übertragung von zumindest einem Datensatz mit den Merkmalen des Anspruchs 1 und durch ein dentales oder dentalchirurgisches Behandlungs- oder Diagnosesystem mit den Merkmalen der Ansprüche 13 und 16 gelöst. Besonders vorteilhafte Ausführungsformen sind jeweils in den Unteransprüchen angeführt.

Das, insbesondere computerimplementierte, Verfahren zur Übertragung von zumindest einem Datensatz zwischen einem dentalen oder dentalchirurgischen Behandlungs- oder Diagnosegerät und einem externen, außerhalb des dentalen oder dentalchirurgischen Behandlungs- oder Diagnosegeräts angeordneten Speicherelement umfasst den Schritt, dass der zumindest eine Datensatz webbasiert mittels Internet und eines Cloud-Computing-Netzwerks, welches das externe Speicherelement aufweist, übertragen wird.

Aufgrund der webbasierten Übertragung des zumindest einen Datensatzes und der Verwendung eines Cloud-Computing-Netzwerks ist es in vorteilhafter Weise möglich, unabhängig von Zeit und Ort und ohne Beschränkung auf eine bestimmte Fernbedienungs-Mensch-Maschine-Schnittstelle auf den zumindest einen Datensatz zugreifen zu können. Auch können vorzugsweise mehrere Personen auf den zumindest einen Datensatz zugreifen.

Das externe Speicherelement ist als Teil des Cloud-Computing-Netzwerks ausgebildet und somit insbesondere entfernt von dem dentalen oder dentalchirurgischen Behandlungs- oder Diagnosegerät und/ oder einer Fernbedienungs-Mensch-Maschine-Schnittstelle angeordnet.

Das externe Speicherelement ist insbesondere dazu ausgebildet, den zumindest einen Datensatz, der webbasiert übertragen wird, zu speichern und/ oder zumindest einen gespeicherten Datensatz zur webbasierten Übertragung an das dentale oder dentalchirurgische Behandlungs- oder Diagnosegerät zur Verfügung zu stellen. Besonders bevorzugt ist das Verfahren zur Übertragung von zumindest einem Datensatz als bidirektionales Verfahren ausgebildet, bei dem Datensätze in beide Richtungen zwischen dem dentalen oder dentalchirurgischen Behandlungs- oder Diagnosegerät und dem externen Speicherelement über das Internet übertragen werden. Durch diese Maßnahmen ist in vorteilhafter Weise ein vollständiger Datenfluss in beide Richtungen möglich.

Der mittels Internet und eines Cloud-Computing-Netzwerks übertragene und/ oder vom externen Speicherelement zu speichernde oder gespeicherte zumindest eine Datensatz umfasst zum Beispiel Betriebs- oder Behandlungsdaten oder zumindest einen Wert zumindest eines Betriebsparameters, die/ der während eines Betriebs oder in Zusammenhang mit einem Betrieb des dentalen oder dentalchirurgischen Behandlungs- oder Diagnosegeräts oder während einer Behandlung oder in Zusammenhang mit einer Behandlung des dentalen oder dentalchirurgischen Behandlungs- oder Diagnosegeräts generiert werden/ wird. Der zumindest eine Datensatz umfasst alternativ oder zusätzlich zum Beispiel Betriebs- oder Behandlungsdaten oder zumindest einen Wert zumindest eines Betriebsparameters, die/ der zum Betrieb des dentalen oder dentalchirurgischen Behandlungs- oder Diagnosegeräts oder für eine Behandlung mit der dentalen oder dentalchirurgischen Behandlungs- oder Diagnosegeräts benötigt werden/ wird und/ oder vor dessen Betrieb oder der Behandlung zur Verfügung gestellt werden/ wird.

Das externe Speicherelement ist vorzugsweise zusätzlich dazu ausgebildet, zumindest einen Datensatz oder zumindest einen Wert zumindest eines Betriebsparameters zu speichern und insbesondere zur webbasierten Übertragung an das dentale oder dentalchirurgische Behandlungs- oder Diagnosegerät zur Verfügung zu stellen, der über eine, im Nachstehenden noch im Detail beschriebene, Fernbedienungs-Mensch-Maschine-Schnittstelle eingebbar ist oder eingegeben wird. Die Fernbedienungs-Mensch-Maschine-Schnittstelle ist vorzugsweise mit dem Cloud-Computing-Netzwerk über das Internet verbunden oder verbindbar. Damit ist es für einen Anwender in vorteilhafter Weise möglich, Datensätze oder Werte individuell einzugeben oder zu ändern.

Der im Vorstehenden genannte, mittels Internet und eines Cloud-Computing-Netzwerks übertragene und/ oder durch das Speicherelement gespeicherte, speicherbare und/ oder zur webbasierten Übertragung zur Verfügung gestellte zumindest eine Datensatz umfasst zum Beispiel technische Daten oder technische Werte eines Parameters, vorzugsweise einen Drehmomentwert, einen Drehmomentgrenzwert, einen Drehzahlwert, einen Drehzahlgrenzwert, ein Übersetzungsverhältnis eines mechanischen Getriebes des dentalen oder dentalchirurgischen Behandlungs- oder Diagnosegeräts, einen Volumenstrom eines Betriebs- oder Kühlmittels, eine Leistung einer Komponente des dentalen oder dentalchirurgischen Behandlungs- oder Diagnosegeräts, zum Beispiel eines Motors oder einer Pumpe, einen Strom- oder Spannungswert einer Komponente des dentalen oder dentalchirurgischen Behandlungs- oder Diagnosegeräts, und ähnliche Daten.

Der zumindest eine Datensatz umfasst alternativ oder zusätzlich zum Beispiel nichttechnische Daten oder Werte, vorzugsweise Angaben über den Anwender des dentalen oder dentalchirurgischen Behandlungs- oder Diagnosegeräts, insbesondere einen Identifikationscode; Angaben über den behandelten Patienten, insbesondere einen Identifikationscode; Angaben über ein ausgewähltes oder benutztes Betriebsprogramm des dentalen oder dentalchirurgischen Behandlungs- oder Diagnosegeräts; Angaben zum dentalen oder dentalchirurgischen Behandlungs- oder Diagnosegerät, insbesondere eine Seriennummer; Angaben zu einem (zu implantierenden oder implantierten) Implantat, insbesondere dessen Hersteller, eine Seriennummer und/ oder das Ablaufdatum; eine Fehlermeldung; oder ähnliche Daten oder Werte.

Vorzugsweise umfasst der zumindest eine Datensatz zumindest Teile einer sogenannten Bibliothek, zum Beispiel einer Implantat-Bibliothek oder einer Werkzeug-Bibliothek, vorzugsweise einer Bibliothek endodontischer Feilen. Eine derartige Bibliothek umfasst insbesondere eine Vielzahl von Daten, Werten und/ oder Spezifizierungen zu verschiedenen Elementen. So umfasst zum Beispiel eine Implantat-Bibliothek Daten oder Werte zu mehreren Implantaten, insbesondere von unterschiedlichen Herstellern. Jedem Implantat der Bibliothek sind dabei vorzugsweise ein oder mehrere spezifische Daten oder Werte zugeordnet, zum Beispiel eine Implantatbezeichnung (Herstellername, Identifizierungscode usw.), die Zahnposition (gemäß Zahnschema), für die das Implantat vorgesehen ist, ein Drehmomentwert, mit dem das Implantat gesetzt werden soll, ein maximaler Drehmomentwert, mit dem das Implantat gesetzt werden darf, ein Übersetzungsverhältnis eines Getriebes eines dentalen Winkelstücks, mit dem das Implantat gesetzt werden soll und/ oder ähnliche Daten und Werte. Das Vorsehen einer derartigen Bibliothek in dem externen Speicherelement vereinfacht einem Anwender in erheblichem Maße die Planung und/ oder Ausführung einer dentalen oder dentalchirurgischen Behandlung, da der Anwender in einfachster Weise Zugriff auf das benötigte Element (Implantat, Werkezeug) und die damit verbundenen Daten und Wert hat. Das Speichern einer Bibliothek in dem externen Speicherelement des Cloud-Computing-Netzwerks hat auch den Vorteil, dass dem Anwender immer die aktuelle Bibliothek zur Verfügung steht, ohne dass diese durch den Anwender gewartet, d.h. ergänzt oder aktualisiert werden muss. Vorzugsweise wird die Bibliothek oder ein Teil davon durch einen Dritten, zum Beispiel einen Hersteller von Implantaten oder Werkzeugen oder des Behandlungs- oder Diagnosegeräts, gewartet, ergänzt und / oder aktualisiert.

Vorzugsweise umfasst der mittels Internet und eines Cloud-Computing-Netzwerks übertragene und/ oder durch das externe Speicherelement speicherbare zumindest eine Datensatz einen oder mehrere dentale oder dentalchirurgische Behandlungspläne, zum Beispiel zur Implantation eines dentalen Implantats oder zu einer endodontischen Behandlung, wobei ein derartiger Behandlungsplan vorzugsweise mehrere der im Vorstehenden genannten Daten, Datensätze und/ oder Parameterwerte umfasst. Damit ist es dem Anwender in vorteilhafter Weise möglich, unabhängig von Zeit und Ort und ohne Beschränkung auf eine bestimmte Fernbedienungs-Mensch-Maschine-Schnittstelle einen Behandlungsplan zu erstellen.

Vorzugsweise umfasst der mittels Internet und eines Cloud-Computing-Netzwerks übertragene und/ oder durch das externe Speicherelement speicherbare zumindest eine Datensatz Angaben für eine Behandlungs- oder Patientendokumentation oder Patientenverwaltung, insbesondere personenbezogen Daten wie zum Beispiel Name, Geschlecht, Alter, Adresse, Kontaktdaten, medizinische Angaben zu Vorerkrankungen, durchgeführte Behandlungen, Diagnosedaten, oder ähnliche Daten. Damit ist es dem Anwender in vorteilhafter Weise möglich, unabhängig von Zeit und Ort und ohne Beschränkung auf eine bestimmte Fernbedienungs-Mensch-Maschine-Schnittstelle auf eine Behandlungs- oder Patientendokumentation oder Patientenverwaltung zuzugreifen.

Das dentale oder dentalchirurgische Behandlungs- oder Diagnosegerät umfasst vorzugsweise zumindest eines der folgenden Elemente: ein, insbesondere schnurlos ausgebildetes, gerades oder gebogenes Handstück, Winkelstück oder Handgriffelement; ein dentales oder dentalchirurgisches Versorgungs- und/ oder Steuergerät, insbesondere ein Tischgerät, zum Betreiben eines dentales oder dentalchirurgisches Handstücks, Winkelstücks und/ oder Instruments; eine Vorrichtung zur Bestimmung der Stabilität eines gesetzten (dentalen) Implantates; eine dentale oder dentalchirurgische Behandlungseinheit mit einem Versorgungs- und/ oder Steuergerät und einem Patientenstuhl.

Das dentale oder dentalchirurgische Behandlungs- oder Diagnosegerät umfasst zumindest eines der folgenden Elemente:
- ein (internes) Speicherelement zum Speichern von zumindest einem Datensatz, insbesondere eines oder mehrerer der im Vorstehenden genannten Datensätze, besonders bevorzugt der Betriebs- oder Behandlungsdaten und/ oder zumindest eines Behandlungsplans; das (interne) Speicherelement ist insbesondere dazu ausgebildet, den zumindest einen Datensatz, der webbasiert mittels Internet und des Cloud-Computing-Netzwerks von dem externen Speicherelement übertragen wird, zu speichern und/ oder zumindest einen Datensatz und/ oder Parameterwert, vorzugsweise einen (insbesondere im Vorstehenden genannten) Betriebs- oder Behandlungswert des Behandlungs- oder Diagnosegeräts, der webbasiert mittels Internet und des Cloud-Computing-Netzwerks an das externe Speicherelement übertragen wird, zwischenzuspeichern und damit in vorteilhafter Weise als Datenpuffer zu dienen, falls die webbasierte Datenübertragung an das Cloud-Computing-Netzwerk unterbrochen ist;
- einen Controller zur Steuerung oder Regelung des Behandlungs- oder Diagnosegeräts und/ oder eines damit verbindbaren Instruments; insbesondere zur Steuerung oder Regelung auf Basis des im Vorstehenden genannten und ggf. im internen Speicher gespeicherten zumindest einen Datensatzes, vorzugsweise auf Basis zumindest einiger der genannten Betriebs- oder Behandlungsdaten, zumindest eines Teils einer Bibliothek und/ oder eines Behandlungsplans, die/ der webbasiert mittels Internet und des Cloud-Computing-Netzwerks von dem externen Speicherelement übertragen werden/ wird;
- eine Anzeigevorrichtung zum Anzeigen von zumindest einem Datensatz oder Parameterwert, insbesondere des im Vorstehenden genannten, webbasiert mittels Internet und des Cloud-Computing-Netzwerks übertragenen zumindest einen Datensatzes, zum Beispiel der Betriebs- oder Behandlungsdaten und/ oder zumindest eines Teils eines Behandlungsplans, sowie von Warnhinweisen, Fehlermeldungen oder ähnlichen Daten;
- eine Stellvorrichtung, zum Beispiel einen Taster, Schieber und/ oder ein Touchscreen, insbesondere zum Stellen zumindest eines Wertes eines Betriebsparameters des dentalen oder dentalchirurgischen Behandlungs- oder Diagnosegeräts und/ oder zum Auswählen eines Betriebsprogrammes oder Behandlungsplans; vorzugsweise ist die Stellvorrichtung ausgebildet, zumindest einen Wert des im Vorstehenden genannten, webbasiert mittels Internet und des Cloud-Computing-Netzwerks übertragenen zumindest einen Datensatzes, vorzugsweise von Betriebs- oder Behandlungsdaten, zumindest eines Parameters und/ oder zumindest eines Behandlungsplans zu stellen oder zu verändern, wodurch in vorteilhafter Weise ein Änderung dieser Parameter auch nach der webbasierten Übertragung möglich ist;
- zumindest einen Sensor zur Detektion eines Messwertes, zum Beispiel einen Stromsensor, Temperatursensor, Drucksensor, Leitfähigkeitssensor oder einen Feuchtigkeitssensor; der Messwert bildet zum Beispiel zumindest einen Teil des im Vorstehenden genannten, insbesondere webbasiert mittels Internet und des Cloud-Computing-Netzwerks übertragenen, Datensatzes, der vorzugsweise im externen Speicherelement des Cloud-Computing-Netzwerks speicherbar ist;
- zumindest eine Medienleitung zur Leitung eines Betriebsmittels oder eines Antriebsmittels, insbesondere von Wasser oder Luft;
- zumindest eine elektrische Leitung zur Versorgung eines elektrischen Verbrauchers mit elektrischer Energie, zum Beispiel eines Elektromotors, einer Strahlungsquelle und/ oder eines Sensors, und/ oder zur Übertragung von Messwerten eines Sensors des dentalen oder dentalchirurgischen Behandlungs- oder Diagnosegeräts;
- eine Werkzeughaltevorrichtung zum, vorzugsweise lösbaren, Halten eines Behandlungswerkzeugs;
- eine Strahlungsquelle, zum Beispiel ein optisches Halbleiterelement, zur Emission von elektromagnetischere Strahlung, insbesondere von sichtbarem Licht und/ oder einer Diagnosestrahlung.

Das Cloud-Computing-Netzwerk umfasst neben dem externen Speicherelement vorzugsweise zumindest weitere Hardware- und/ oder Softwareelemente, zum Beispiel eines der folgenden Elemente: einen Prozessor; einen Mikrocontroller, insbesondere mit einem oder mehreren elektronischen Schaltkreisen; ein oder mehrere, insbesondere durch zumindest einen elektronischen Schaltkreis des Mikrocontrollers ausführbare, Softwareprogramme oder Webapplikationen, insbesondere zum Speichern, Empfangen, Verarbeiten, Versenden, Verschlüsseln, Entschlüsseln und/ oder zum Verfügung stellen des über das Internet übertragenen zumindest einen Datensatzes oder Parameterwerts. Die Webapplikation ist insbesondere über die Fernbedienungs-Mensch-Maschine-Schnittstelle aufrufbar und/ oder bedienbar. Der Mikrocontroller und das externe Speicherelement sind insbesondere elektrisch miteinander verbunden, so dass der Mikrocontroller auf die in dem externen Speicherelement gespeicherten Datensätze und/ oder Software zugreifen kann.

Vorzugsweise ist vorgesehen, dass der zumindest eine (übertragene und/ oder im externen Speicher gespeicherte) Datensatz über eine mit dem Cloud-Computing-Netzwerk kommunikativ verbundene oder verbindbare Fernbedienungs-Mensch-Maschine-Schnittstelle angezeigt und/ oder aufgerufen und/ oder bearbeitet wird. Es ist alternativ oder zusätzlich auch möglich, dass ein (weiterer) Datensatz über die Fernbedienungs-Mensch-Maschine-Schnittstelle eingegeben und vorzugsweise in dem externen Speicher gespeichert wird. Vorzugsweise ist die Fernbedienungs-Mensch-Maschine-Schnittstelle kommunikativ (über das Cloud-Computing-Netzwerk und das Internet) auch mit dem Behandlungs- oder Diagnosegerät verbunden. Die kommunikative Verbindung der Fernbedienungs-Mensch-Maschine-Schnittstelle mit dem Cloud-Computing-Netzwerk kann drahtgebunden und/ oder drahtlos erfolgen oder ausgebildet sein.

Vorzugsweise umfasst die Fernbedienungs-Mensch-Maschine-Schnittstelle zum Beispiel ein mobiles elektronisches Gerät, insbesondere eine Mobiltelefon, einen tragbaren Computer, einen Laptop, einen Tablet-Computer oder ähnliche Geräte, oder ein stationäres elektronisches Gerät, zum Beispiel einen Personalcomputer.

Vorzugsweise wird über die Fernbedienungs-Mensch-Maschine-Schnittstelle eine in dem Cloud-Computing-Netzwerk gespeicherte Webapplikation aufgerufen, um den in dem externen Speicherelement gespeicherten zumindest einen Datensatz aufzurufen und/ oder anzuzeigen und/ oder zu bearbeiten und/ oder zumindest einen Datensatz einzugeben und in dem externen Speicher zu speichern. Vorzugsweise umfasst die Fernbedienungs-Mensch-Maschine-Schnittstelle einen Webbrowser, um auf die Webapplikation zugreifen zu können.

Vorzugsweise wird der (übertragene und/ oder gespeicherte) zumindest eine Datensatz durch einen Mikrocontroller des Cloud-Computing-Netzwerks verarbeitet. Vorzugsweise umfasst das Cloud-Computing-Netzwerk Software und/ oder Hardware zum Bearbeiten oder Verarbeiten des zumindest einen Datensatzes, zum Beispiel Software zur (grafischen) Darstellung des zumindest einen Datensatzes, zur Verknüpfung des zumindest einen Datensatzes mit bereits gespeicherten Daten und/ oder zum Vergleichen verschiedener Datensätze. Damit vergrößert sich in vorteilhafter Weise die Funktionalität des Behandlungs- oder Diagnosegeräts.

Vorzugsweise umfasst oder speichert das externe Speicherelement Software zur Planung einer Behandlung oder Diagnose mit dem dentalen oder dentalchirurgischen Behandlungs- oder Diagnosegerät, wobei der webbasiert mittels Internet und eines Cloud-Computing-Netzwerks übertragene zumindest eine Datensatz einen mit der Software erstellten Behandlungsplan umfasst. Vorzugsweise umfasst der Behandlungsplan einen oder mehrere Behandlungs- oder Diagnoseschritte. Vorzugsweise umfasst der Behandlungsplan zumindest einen der im Vorstehenden genannten Betriebs- oder Behandlungsdaten oder zumindest einen Wert zumindest eines Betriebsparameters. Vorzugsweise werden die Betriebs- oder Behandlungsdaten oder der zumindest einen Wert durch den Anwender eingegeben oder aus voreingestellten Werten ausgewählt. Vorzugsweise erstellt ein Anwender einen oder mehrere Behandlungspläne und sendet zumindest einen dieser Behandlungspläne an eines oder mehrere Behandlungs- oder Diagnosegeräte.

Vorzugsweise erstellt ein Anwender mehrere Behandlungspläne für mehrere Behandlungen oder Diagnosen, zum Beispiel dentalchirurgischer Implantationen, die er auf demselben dentalen oder dentalchirurgischen Behandlungs- oder Diagnosegerät durchführen möchte. Vorzugsweise sendet der Anwender diesen zumindest einen Behandlungsplan, insbesondere in Form eines (einzigen) Datenpakets oder Datensatzes, an das eine Behandlungs- oder Diagnosegerät, welches die mehreren Behandlungspläne in einem internen Speicher speichert. Vorzugsweise führt der Anwender die mehreren Behandlungen oder Diagnosen nacheinander auf Basis der übertragenen Behandlungspläne durch. Vorzugsweise ändert der Anwender vor Durchführung einer Behandlung oder Diagnose zumindest einen Wert oder Angabe zumindest in einem der übertragenen Behandlungspläne, insbesondere mit einer im Vorstehenden beschriebenen Stellvorrichtung des dentalen oder dentalchirurgischen Behandlungs- oder Diagnosegeräts. Das Erstellen mehrerer Behandlungspläne für mehrere Behandlungen oder Diagnosen und vorzugsweise das Senden mehrerer Behandlungspläne an ein (einziges) Behandlungs- oder Diagnosegerät in einem einzigen Sendevorgang erhöht in vorteilhafter Weise die Anwenderfreundlichkeit, da ein Anwender nicht mehr gezwungen ist, jeden Behandlungsplan einzeln, insbesondere über die Stellvorrichtung des dentalen oder dentalchirurgischen Behandlungs- oder Diagnosegeräts, zu erstellen.

Vorzugsweise wird der zumindest eine Datensatz vor der webbasierten Übertragung mittels Internet und eines Cloud-Computing-Netzwerks verschlüsselt und nach der webbasierten Übertragung entschlüsselt. Dazu weisen das dentalen oder dentalchirurgischen Behandlungs- oder Diagnosegerät und das Cloud-Computing-Netzwerk jeweils Software zum Ver- und/ oder Entschlüsseln auf. Damit ist in vorteilhafter Weise eine gesicherte Übertragung des zumindest einen Datensatzes, insbesondere der darin enthaltenen Patientendaten, gewährleistet.

Ein bevorzugtes (computerimplementiertes) Verfahren zur Erstellung eines Behandlungsplans für eine dentalchirurgische Implantation, bei dem zumindest ein Datensatz zwischen einem dentalchirurgischen Behandlungsgerät und einem externen, außerhalb des dentalchirurgischen Behandlungsgeräts angeordneten Speicherelement webbasiert mittels Internet und eines Cloud-Computing-Netzwerks, welches das externe Speicherelement umfasst, übertragen wird, umfasst folgende Schritte: (A) zur Verfügung stellen einer Fernbedienungs-Mensch-Maschine-Schnittstelle, die kommunikativ mit dem Cloud-Computing-Netzwerk verbunden ist oder wird; (B) Aufrufen einer in dem Speicherelement des Cloud-Computing-Netzwerks gespeicherten Webapplikation zur Erstellung eines Behandlungsplans einer dentalchirurgischen Implantation mit der Fernbedienungs-Mensch-Maschine-Schnittstelle; (C) Eingabe und/ oder Auswahl und/ oder Änderung zumindest eines Datensatzes und/ oder Parameterwertes für die dentalchirurgischen Implantation über die aufgerufene Webapplikation und Übernahme oder Speicherung dieses Datensatzes und/ oder Parameterwertes in den Behandlungsplan; und (D) webbasiertes Übertragen des Behandlungsplans (einschließlich des zumindest einen Datensatzes und/ oder Parameterwertes) mittels Internet von dem Cloud-Computing-Netzwerk auf das dentalchirurgische Behandlungsgerät, insbesondere auf ein (internes) Speicherelement des dentalchirurgischen Behandlungsgeräts.

Damit ist es dem Anwender in vorteilhafter Weise möglich, unabhängig von Zeit und Ort und ohne Beschränkung auf eine bestimmte Fernbedienungs-Mensch-Maschine-Schnittstelle einen Behandlungsplan für eine dentalchirurgische Implantation zu erstellen.

Vorzugsweise speichert das Speicherelement des Cloud-Computing-Netzwerks eine Implantatbibliothek, insbesondere eine im Vorstehenden beschriebene Implantatbibliothek. Vorzugsweise umfasst der im Vorstehenden beschriebene Verfahrensschritt (C), dass über die Webapplikation und die Fernbedienungs-Mensch-Maschine-Schnittstelle ein Implantat der Implantatbibliothek ausgewählt wird, insbesondere ein Implantat, das in der dentalchirurgischen Implantation implantiert wird. Die Auswahl des Implantats erflogt zum Beispiel über die Implantatbezeichnung und/ oder die Zahnposition (gemäß Zahnschema), für die das Implantat in der Implantatbibliothek vorgesehen ist/ sind. Besonders bevorzugt wird durch die Auswahl des Implantats zumindest ein, dem Implantat zugeordneter Datensatz und/ oder Behandlungswert, zum Beispiel ein Drehmomentwert, mit dem das Implantat gesetzt werden soll, ein maximaler Drehmomentwert, mit dem das Implantat gesetzt werden darf, und/ oder ein Übersetzungsverhältnis eines Getriebes eines dentalen Winkelstücks, mit dem das Implantat gesetzt werden soll, ausgewählt und (automatisch) in den Behandlungsplan eingetragen oder übernommen. Das Vorsehen einer Implantatbibliothek vereinfacht somit erheblich die Erstellung eines Behandlungsplans.

Vorzugsweise speichert das Speicherelement des Cloud-Computing-Netzwerks eine Software oder eine Datenbank für eine Patienten- oder Behandlungsdokumentation oder für eine Patientenverwaltung, insbesondere mit Angaben zu einem Patienten, wie dies im Vorstehenden beschrieben ist. Vorzugsweise umfasst das im Vorstehenden beschriebene Verfahren zur Erstellung eines Behandlungsplans für eine dentalchirurgische Implantation den zusätzlichen Schritt, dass der Behandlungsplan und Angaben zu dem (zu behandelnden) Patienten und/ oder Angaben zu einem (zu implantierenden) Implantat, insbesondere aus der genannten Software oder Datenbank, verknüpft werden und/ oder der Behandlungsplan und/ oder Behandlungsdaten in der genannten Software oder Datenbank gespeichert wird/ werden. Das Vorsehen einer Software oder einer Datenbank für eine Patienten- oder Behandlungsdokumentation oder für eine Patientenverwaltung vereinfacht somit erheblich die Dokumentation einer dentalchirurgischen Behandlung.

Vorzugsweise ist des Weiteren vorgesehen, dass das dentalchirurgische Behandlungsgerät einen Controller zur Steuerung oder Regelung des dentalchirurgischen Behandlungsgerät und/ oder eines damit verbindbaren dentalchirurgischen Instruments aufweist. Vorzugsweise wird die dentalchirurgische Implantation und/ oder das dafür benötigte Instrument, zum Beispiel ein dentalchirurgisches Winkelstück, durch den Controller auf Basis des webbasiert übertragenen (und im internen Speicher gespeicherten) Behandlungsplans gesteuert. Damit erspart sich der Anwender die mühsame Einstellung der benötigten Betriebsparameter am dentalchirurgischen Behandlungsgerät mittels Tasten und ähnlichen Bedienelementen.

Vorzugsweise umfasst das im Vorstehenden beschriebene Verfahren zur Erstellung eines Behandlungsplans für eine dentalchirurgische Implantation die Erstellung mehrerer Behandlungspläne, insbesondere für unterschiedliche Patienten, gemäß den Schritten (A) - (C) und das webbasierte Übertragen dieser mehreren Behandlungspläne gemäß Schritt (D) auf ein oder mehrere dentalchirurgische Behandlungsgeräte, insbesondere auf dessen/ deren (internes) Speicherelement. Vorzugsweise werden die mehreren Behandlungspläne als ein Datensatz oder Datenpaket an das dentalchirurgische Behandlungsgerät gesendet. Dies erleichtert die Erstellung der Behandlungspläne erheblich, da ein Anwender mehrere Behandlungspläne, zum Beispiel jene für den gesamten kommenden Tag, auf einmal erstellen und übertragen kann.

Bei einem anderen bevorzugten (computerimplementierten) Verfahren zur webbasierten Übertragung von zumindest einem Datensatz zwischen einem dentalchirurgischen Behandlungsgerät und einem externen, außerhalb des dentalchirurgischen Behandlungsgeräts angeordneten Speicherelement mittels Internet und eines Cloud-Computing-Netzwerks, welches das externe Speicherelement aufweist, umfasst der zumindest eine Datensatz Mess-, Betriebs- oder Behandlungsdaten, die für eine dentalchirurgische Implantation benötigt werden, insbesondere Steuerdaten oder Steuerwerte wie zumindest ein Drehmoment- oder Drehzahlwert, wobei der zumindest eine Datensatz von dem Cloud-Computing-Netzwerk, insbesondere dem externen Speicherelement, an das dentalchirurgische Behandlungsgerät über das Internet gesendet wird. Damit ist es dem Anwender in vorteilhafter Weise möglich, unabhängig von Zeit und Ort und ohne Einschränkung auf ein dentalchirurgisches Behandlungsgerät Werte für eine Implantation festzulegen.

Bei einem weiteren bevorzugten (computerimplementierten) Verfahren zur webbasierten Übertragung von zumindest einem Datensatz zwischen einem dentalchirurgischen Behandlungsgerät und einem externen, außerhalb des dentalchirurgischen Behandlungsgeräts angeordneten Speicherelement mittels Internet und eines Cloud-Computing-Netzwerks, welches das externe Speicherelement aufweist, umfasst der zumindest eine Datensatz Mess-, Betriebs- oder Behandlungsdaten, die bei einer dentalchirurgischen Implantation gemessen, detektiert und / oder generiert wurden, wobei der zumindest eine Datensatz von dem dentalchirurgischen Behandlungsgerät über das Internet an das Cloud-Computing-Netzwerk, insbesondere das externe Speicherelement, gesendet wird. Damit ist es dem Anwender in vorteilhafter Weise möglich, unabhängig von Zeit und Ort und ohne Beschränkung auf eine bestimmte Fernbedienungs-Mensch-Maschine-Schnittstelle den zumindest einen Datensatz aufzurufen.

Die Mess-, Betriebs- oder Behandlungsdaten dieses Verfahrens umfassen generell zumindest einen der im Vorstehenden genannten Daten oder Werte, vorzugsweise die genannten technischen Daten oder technische Werte eines Parameters und/ oder die genannten nicht-technischen Daten oder Werte. Besonders bevorzugt umfassen die Mess-, Betriebs- oder Behandlungsdaten einen oder mehrere Drehmomentwerte, die während des Setzen des Implantats gemessen wurden, insbesondere durch einen im Vorstehenden genannten Sensor des dentalchirurgischen Behandlungsgeräts, oder eine aus den einzelnen, gemessenen Drehmomentwerten generierte Drehmomentkurve. Vorzugsweise umfassen die Mess-, Betriebs- oder Behandlungsdaten eine Zeit- und/ oder Datumsangabe, insbesondere zum Nachweis, wann die dentalchirurgischen Implantation stattgefunden hat und/ oder wann die Mess-, Betriebs- oder Behandlungsdaten generiert wurden und/ oder versendet wurde.

Bei einem anderen bevorzugten (computerimplementierten) Verfahren zur webbasierten Übertragung von zumindest einem Datensatz zwischen einem dentalen oder dentalchirurgischen Behandlungsgerät und einem externen, außerhalb des dentalen oder dentalchirurgischen Behandlungsgeräts angeordneten Speicherelement mittels Internet und eines Cloud-Computing-Netzwerks, welches das externe Speicherelement aufweist, umfasst der zumindest eine Datensatz zumindest einen Messwert der Stabilität eines in einem Knochen fixierten (dentalen) Implantats, wobei der zumindest eine Datensatz von dem dentalchirurgischen Behandlungsgerät über das Internet an das Cloud-Computing-Netzwerk, insbesondere das externe Speicherelement, gesendet wird. Damit ist es dem Anwender in vorteilhafter Weise möglich, unabhängig von Zeit und Ort und ohne Beschränkung auf eine bestimmte Fernbedienungs-Mensch-Maschine-Schnittstelle auf den zumindest einen Messwert der Implantatstabilität Zugriff zu haben.

Vorzugsweise umfasst der zumindest eine Datensatz, insbesondere der Datensatz mit den Mess-, Betriebs- oder Behandlungsdaten oder dem Messwert der Implantatstabilität, einen Wert oder eine Angabe, der/ die vor der dentalen oder dentalchirurgischen Behandlung oder Diagnose, zum Beispiel Implantation, an das dentale oder dentalchirurgische Behandlungsgerät übertragen wurde, insbesondere webbasiert mittels Internet und des Cloud-Computing-Netzwerks, welches das externe Speicherelement umfasst. Beispielsweise handelt es sich dabei um Daten zur Identifikation des Patienten, die insbesondere mit einem Behandlungsplan vor der dentalchirurgischen Implantation an das dentalchirurgischen Behandlungsgerät übertragen wurden. Damit ist in vorteilhafter Weise ein exakte Zuordnung des zumindest einen Datensatzes möglich.

Vorzugsweise umfasst das Verfahren zur webbasierten Übertragung von zumindest einem Datensatz den weiteren Schritt, dass der zumindest eine Datensatz, insbesondere der Datensatz mit den Mess-, Betriebs- oder Behandlungsdaten oder dem Messwert der Implantatstabilität, vor der webbasierten Übertragung an das dentale oder dentalchirurgische Behandlungs- oder Diagnosegerät in einem internen Speicherelement des dentalen oder dentalchirurgischen Behandlungs- oder Diagnosegeräts und/ oder in einem Speicherelement einer Sende- und Empfangsvorrichtung, welche die Mess-, Betriebs- oder Behandlungsdaten webbasiert überträgt, zwischengespeichert wird. Die Sende- und Empfangsvorrichtung ist zum Beispiel als Router oder Gateway ausgebildet. Die genannten Speicherelemente bilden in vorteilhafter Weise einen Datenpuffer, um insbesondere den Verlust von Daten zu verhindern, wenn zum Beispiel die webbasierte Datenübertragung an das Cloud-Computing-Netzwerk unterbrochen ist.

Vorzugsweise umfasst das Verfahren zur webbasierten Übertragung von zumindest einem Datensatz den weiteren Schritt, dass eine Fernbedienungs-Mensch-Maschine-Schnittstelle, die kommunikativ mit dem Cloud-Computing-Netzwerk verbunden ist oder wird, zur Verfügung gestellt wird und dass insbesondere der zumindest eine Datensatz, der von dem dentalen oder dentalchirurgischen Behandlungs- oder Diagnosegerät über das Internet an das Cloud-Computing-Netzwerk, insbesondere das externe Speicherelement, gesendet wurde, über die Fernbedienungs-Mensch-Maschine-Schnittstelle angezeigt und/ oder aufgerufen und/ oder bearbeitet wird. Der übertragene Datensatz kann auch ausgedruckt und/ oder an ein anderes elektronisches Gerät weitergeleitet werden. Aufgrund der Speicherung des zumindest einen Datensatzes in dem Cloud-Computing-Netzwerk ist es in vorteilhafter Weise möglich, unabhängig von Zeit und Ort und ohne Beschränkung auf eine bestimmte Fernbedienungs-Mensch-Maschine-Schnittstelle auf diesen Datensatz zugreifen zu können.

Vorzugsweise speichert das Speicherelement des Cloud-Computing-Netzwerks eine Software oder eine Datenbank für eine Patienten- oder Behandlungsdokumentation oder für eine Patientenverwaltung, insbesondere mit Angaben zu einem Patienten, wie dies im Vorstehenden beschrieben ist. Vorzugsweise umfasst das im Vorstehenden beschriebene Verfahren zur webbasierten Übertragung von zumindest einem Datensatz den weiteren Schritt, dass der zumindest eine übertragene Datensatz und Angaben zu dem (behandelten) Patienten, insbesondere aus der genannten Software oder Datenbank, verknüpft werden und/ oder der zumindest eine übertragene Datensatz in der Software oder Datenbank für eine Patienten- oder Behandlungsdokumentation oder für eine Patientenverwaltung gespeichert wird. Die Verknüpfung oder Speicherung erfolgt vorzugsweise automatisch, insbesondere auf Basis von Daten zur Identifikation des Patienten, die in dem zumindest einen webbasiert übertragenen Datensatz enthalten sind. Dieser Verfahrensschritt vereinfacht somit erheblich die Dokumentation einer dentalchirurgischen Behandlung.

Vorzugsweise umfasst das Verfahren zur webbasierten Übertragung von zumindest einem Datensatz den weiteren Schritt, dass der Datensatz, insbesondere der Datensatz mit den Mess-, Betriebs- oder Behandlungsdaten oder dem Messwert der Implantatstabilität, vor der webbasierten Übertragung an das Cloud-Computing-Netzwerk mit einer Datums- und/ oder Zeitangabe (Datums- oder Zeitstempel) versehen wird. Vorzugsweise ist die Sende- und Empfangsvorrichtung des dentalen oder dentalchirurgischen Behandlungs- oder Diagnosegerät ausgebildet, die Datums- und/ oder Zeitangabe dem Datensatz hinzuzufügen. Vorzugsweise weist die Sende- und Empfangsvorrichtung dafür einen eigenen elektronischen Datums- und/ oder Zeitmesser (Timer und/ oder Controller) auf oder sie ist ausgebildet, auf einen online-Datums- und/ oder Zeitgeber zuzugreifen. Die Sende- und Empfangsvorrichtung ist zum Beispiel als Router oder Gateway ausgebildet. Das Hinzufügen einer Datums- und/ oder Zeitangabe erleichtert in vorteilhafter Weise die zeitliche Dokumentation der Behandlung oder Diagnose.

Das Hinzufügen einer Datums- und/ oder Zeitangabe ist insbesondere bei dem Verfahren, bei dem der zumindest eine Datensatz zumindest einen Messwert der Stabilität eines in einem Knochen fixierten Implantats enthält, von großem Vorteil. Vorzugsweise umfasst das Verfahren des Weiteren, dass mehrere Messwert der Implantatstabilität in zeitlichem Abstand, insbesondere von mehreren Tagen, Wochen oder Monaten, ermittelt werden. Vorzugsweise wird jedem dieser Datensätze mit den weiteren Stabilitätsmesswerten eine Datums- und/ oder Zeitangabe hinzugefügt. Vorzugsweise werden die Datensätze mit dem Stabilitätsmesswert und der Datums- und/ oder Zeitangabe in dem externen Speicherelement des Cloud-Computing-Netzwerks gespeichert. Vorzugsweise werden die übertragenen Datensätze mit dem Stabilitätsmesswert und der Datums- und/ oder Zeitangabe und Angaben zu dem (behandelten) Patienten, insbesondere aus der Software oder Datenbank für eine Patienten- oder Behandlungsdokumentation oder für eine Patientenverwaltung, verknüpft und/ oder in der genannten Software oder Datenbank gespeichert. Vorzugsweise weist das Cloud-Computing-Netzwerk eine Software zur (automatischen) Darstellung der Datensätze mit dem Stabilitätsmesswert und der jeweiligen (zugehörigen) Datums- und/ oder Zeitangabe eines Patienten auf, insbesondere zur gemeinsamen (grafischen) Darstellung der Datensätze in einem Diagramm, vorzugsweise als Diagramm, in dem die gemessenen Stabilitätsmesswerte in Relation zu dem zeitlichen Verlauf (basierend auf den jeweiligen Datums- und/ oder Zeitangaben der Datensätze) dargestellt sind. Vorzugsweise ist diese Darstellung über die Fernbedienungs-Mensch-Maschine-Schnittstelle anzeigbar und/ oder aufrufbar. Damit erhält der Anwender in vorteilhafter Weise einen zeitlichen Überblick über den Verlauf der Implantatstabilität, insbesondere über den Verlauf des Einheilens eines kürzlich gesetzten Implantats.

Bei diesem genannten Verfahren umfasst das dentalen oder dentalchirurgischen Behandlungs- oder Diagnosegerät vorzugsweise eine Vorrichtung zur Bestimmung der Stabilität eines gesetzten Implantates, insbesondere zur Bestimmung der Qualität des Einheilens des Implantats oder der Verbindung des Implantats mit der umgebenden Knochensubstanz. Eine derartige Vorrichtung zur Bestimmung der Implantatstabilität ist insbesondere dazu ausgebildet, das Implantat berührungslos oder kontaktierend in Schwingung zu versetzen, zum Beispiel magnetisch oder mittels Schallwellen. Die Vorrichtung ist des Weiteren dazu ausgebildet, ein Antwortsignal des Implantats, insbesondere eine Schwingung, zu empfangen und zu verarbeiten, auf dessen Basis, zum Beispiel der Schwingungsfrequenz, ein Messwert der Stabilität eines in einem Knochen fixierten Implantats ermittelbar ist.

Gemäß einem weiteren (computerimplementierten) Verfahren zur Erstellung eines Implantatpasses oder Implantatausweises (für einen Patienten) werden Daten eines (zu implantierenden oder implantierten) Implantats in dem externen Speicherelement des Cloud-Computing-Netzwerks gespeichert, insbesondere in einer Software oder einer Datenbank für eine Patienten- oder Behandlungsdokumentation oder für eine Patientenverwaltung des externen Speicherelements. Die Daten des Implantats umfassend zum Beispiel zumindest eine der folgenden Angaben: Angaben zum Hersteller des Implantats, insbesondere Name, Adresse, Kontaktdaten, Internetseite; eine Seriennummer des Implantats; einen Namen und/ oder Typ und/ oder ein Modell des Implantats; ein Ablaufdatum bis zu dem das Implantat implantiert werden muss; Hinweise für den Empfänger oder Träger des Implantats; oder ähnliche Angaben. Vorzugsweise werden die Daten des Implantats mit einer Bildaufnahmevorrichtung, insbesondere einer Kamera, aufgenommen, insbesondere von einem Datenspeicher, zum Beispiel einem Barcode, auf der Verpackung des Implantats. Alternativ werden die Daten des Implantats mit einer Lesevorrichtung aus einem elektronischen Datenspeicher, zum Beispiel einem RFID-Tag, auf der Verpackung des Implantats ausgelesen. Vorzugsweise werden die Daten eines (zu implantierenden oder implantierten) Implantats mit einer oder mehreren Angaben zu dem Patienten, i.e. dem Empfänger des Implantats, verknüpft, wobei die Angaben zu dem Patienten besonders bevorzugt aus der Software oder einer Datenbank für eine Patienten- oder Behandlungsdokumentation oder für eine Patientenverwaltung des externen Speicherelements aufgerufen werden.

Vorzugsweise werden des Weiteren über eine Fernbedienungs-Mensch-Maschine-Schnittstelle, die kommunikativ mit dem Cloud-Computing-Netzwerk verbunden ist oder wird, die Daten des Implantats angezeigt und/ oder aufgerufen. Insbesondere wird über die Fernbedienungs-Mensch-Maschine-Schnittstelle der Befehl zum Ausdrucken der Daten des Implantats, vorzugsweise zusätzlich mit zumindest einer Angaben zu dem Patienten, und somit zum Erstellen des Implantatpasses oder Implantatausweises erteilt.

Vorzugsweise ist das Verfahren zur Erstellung eines Implantatpasses oder Implantatausweises mit einem der im Vorstehenden (computerimplementierten) Verfahren zur webbasierten Übertragung von zumindest einem Datensatz zwischen einem dentalen oder dentalchirurgischen Behandlungsgerät und einem externen, außerhalb des Behandlungsgeräts angeordneten Speicherelement mittels Internet und eines Cloud-Computing-Netzwerks, welches das externe Speicherelement aufweist, kombinierbar oder integrierbar oder als Teil eines derartigen Verfahren zur webbasierten Übertragung von zumindest einem Datensatz ausgebildet.

Es ist des Weiteren ein Computerprogrammprodukt vorgesehen, das Befehle umfasst, die bei der Ausführung des Programms durch einen Computer diesen veranlassen, ein im Vorstehenden genanntes oder beschriebenes Verfahren auszuführen. Das Computerprogrammprodukt beinhaltet insbesondere zumindest einen maschinenlesbaren Programmträger, auf dem ein Computer-Programm oder Software in der Form von elektronisch und/oder optisch lesbaren Befehlen oder Steuersignalen zum Ausführen eines im Vorstehenden genannten oder beschriebenen Verfahrens gespeichert ist.

Es ist des Weiteren auch ein computerlesbares Speichermedium vorgesehen, das Befehle umfasst, die bei der Ausführung durch einen Computer diesen veranlassen, ein im Vorstehenden genanntes oder beschriebenes Verfahren auszuführen.

Ein dentales oder dentalchirurgisches Behandlungs- oder Diagnosesystem umfasst ein dentales oder dentalchirurgisches Behandlungs- oder Diagnosegerät, ein Cloud-Computing-Netzwerk mit einem externen, außerhalb des dentalen oder dentalchirurgischen Behandlungs- oder Diagnosegeräts angeordneten Speicherelement und eine Vorrichtung zur Datenverarbeitung mit Mitteln zur Ausführung eines der im Vorstehenden genannten oder beschriebenen Verfahren. Das dentale oder dentalchirurgische Behandlungs- oder Diagnosegerät umfasst insbesondere zumindest eines der im Vorstehenden genannten Behandlungs- oder Diagnosegeräte. Vorzugsweise umfasst das Cloud-Computing-Netzwerk zumindest einen Teil der Vorrichtung zur Datenverarbeitung. Insbesondere bildet ein Mikrocontroller des Cloud-Computing-Netzwerks zumindest einen Teil der Vorrichtung zur Datenverarbeitung.

Ein anderes dentales oder dentalchirurgisches Behandlungs- oder Diagnosesystem umfasst ein dentales oder dentalchirurgisches Behandlungs- oder Diagnosegerät und eine Sende- und Empfangsvorrichtung zur kommunikativen Verbindung mit einer Fernbedienungs-Mensch-Maschine-Schnittstelle, wobei die Sende- und Empfangsvorrichtung ausgebildet ist, eine webbasierte Anbindung über das Internet und ein Cloud-Computing-Netzwerk an die Fernbedienungs-Mensch-Maschine-Schnittstelle bereitzustellen. Das dentale oder dentalchirurgische Behandlungs- oder Diagnosegerät umfasst insbesondere zumindest eines der im Vorstehenden genannten Behandlungs- oder Diagnosegeräte.

Die dentalen oder dentalchirurgischen Behandlungs- oder Diagnosesysteme ermöglichen einem Anwender in vorteilhafter Weise unabhängig von Zeit und Ort und ohne Beschränkung auf eine bestimmte Fernbedienungs-Mensch-Maschine-Schnittstelle auf einen in dem Cloud-Computing-Netzwerk, insbesondere in dessen externen Speicherelement, gespeicherten Datensatz zugreifen zu können.

Die dentalen oder dentalchirurgischen Behandlungs- oder Diagnosesysteme umfassen vorzugsweise eine Sende- und Empfangsvorrichtung, insbesondere einen Router oder ein Gateway. Vorzugsweise ist die Sende- und Empfangsvorrichtung, insbesondere das Gateway, ausgebildet, drahtlos mit dem dentalen oder dentalchirurgischen Behandlungs- oder Diagnosegerät zu kommunizieren. Vorzugsweise ist die Sende- und Empfangsvorrichtung, insbesondere das Gateway, ausgebildet, drahtlos mit einem lokalen Netzwerk, insbesondere einem lokalen Netzwerk in einer dentalen oder dentalchirurgischen Praxis, zu kommunizieren. Vorzugsweise ist die Sende- und Empfangsvorrichtung, insbesondere das Gateway, drahtgebunden mit dem Cloud-Computing-Netzwerk verbunden oder verbindbar. Damit ist in vorteilhafter Weise ein variables System zur Übertragung des zumindest einen Datensatzes zwischen dem dentalen oder dentalchirurgischen Behandlungs- oder Diagnosegerät und dem externen Speicherelement des Cloud-Computing-Netzwerks geschaffen, nämlich entweder drahtgebunden oder drahtlos über das lokale Netzwerk, das an das Internet angeschlossen ist.

Vorzugsweise ist die Sende- und Empfangsvorrichtung, insbesondere das Gateway, ausgebildet, einen Datensatz (insbesondere mit einem Wert zur Messung der Stabilität eines in einem Knochen fixierten Implantats), der über das Gateway übertragen wird, mit einer Datums- und/ oder Zeitangabe zu versehen, so wie dies im Vorstehenden bereits beschrieben ist.

Die Sende- und Empfangsvorrichtung ist entweder als separates Element, das mit dem dentalen oder dentalchirurgischen Behandlungs- oder Diagnosegerät zumindest kommunikativ verbindbar ist, oder als in das Behandlungs- oder Diagnosegerät integriertes Element ausgebildet.

Vorzugsweise weist das dentalen oder dentalchirurgischen Behandlungs- oder Diagnosegerät Software zum (drahtlosen) Pairing mit der Sende- und Empfangsvorrichtung auf.

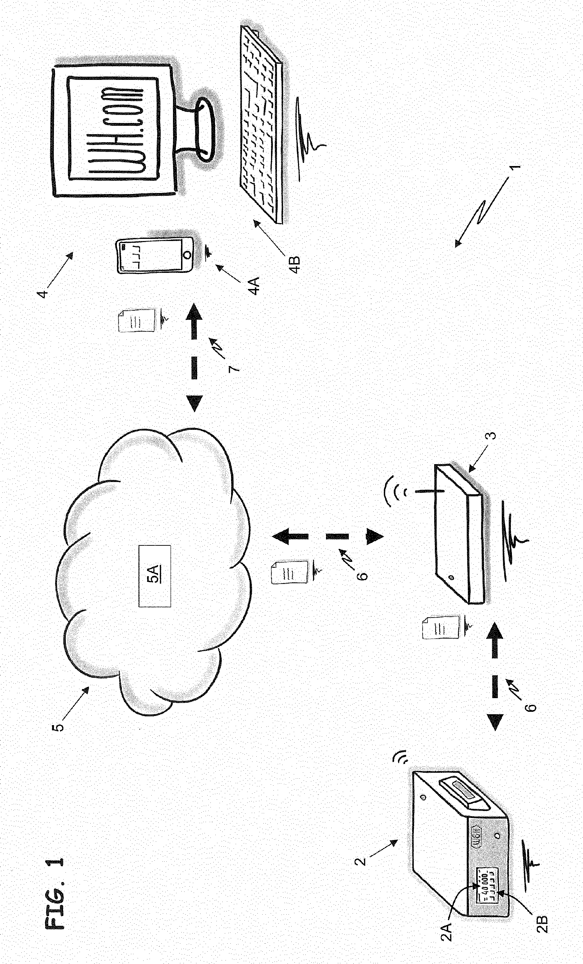

Die Erfindung wird nachfolgend anhand eines bevorzugten Ausführungsbeispiels und Bezug nehmend auf die beigefügte Zeichnung erläutert. Es zeigt die einzige Figur 1 ein dentales oder dentalchirurgisches Behandlungs- oder Diagnosesystem mit webbasierter Übertragung eines Datensatzes.

Das Behandlungs- oder Diagnosesystem 1 umfasst ein dentales oder dentalchirurgisches Behandlungs- oder Diagnosegerät 2 und eine Sende- und Empfangsvorrichtung 3 zur kommunikativen Verbindung mit einer Fernbedienungs-Mensch-Maschine-Schnittstelle 4. Die Sende- und Empfangsvorrichtung 3 ist ausgebildet, eine webbasierte Anbindung des dentalen oder dentalchirurgischen Behandlungs- oder Diagnosegerät 2 über das Internet und ein Cloud-Computing-Netzwerk 5 an die Fernbedienungs-Mensch-Maschine-Schnittstelle bereitzustellen.

Das dentale oder dentalchirurgische Behandlungs- oder Diagnosegerät 2 ist in der Figur 1 als dentalchirurgisches Tischgerät, insbesondere zur Durchführung dentalchirurgischer Eingriffe, vorzugsweise Implantationen, dargestellt. Es sei jedoch ausdrücklich noch einmal darauf verwiesen, dass das dentale oder dentalchirurgische Behandlungs- oder Diagnosegerät 2 eine Vielzahl anderer Geräte, zum Beispiel ein Handstück, ein Winkelstück, eine Vorrichtung zur Bestimmung der Stabilität eines gesetzten Implantates oder eine dentale oder dentalchirurgische Behandlungseinheit umfassen kann.

Das dentale oder dentalchirurgische Behandlungs- oder Diagnosegerät 2 umfasst eine Anzeigevorrichtung 2A zum Anzeigen von Daten, insbesondere auch von Werten, Parametern und/ oder Angaben des zumindest einen webbasiert übertragenen Datensatzes. Das dentale oder dentalchirurgische Behandlungs- oder Diagnosegerät 2 umfasst zumindest ein Stellelement 2B, um Werte, Parameter und/ oder Angaben, insbesondere des zumindest einen webbasiert übertragenen Datensatzes, verändern zu können.

Über eine webbasierte Verbindung 6 zwischen dem Cloud-Computing-Netzwerk 5 und dem dentalen oder dentalchirurgischen Behandlungs- oder Diagnosegerät 2 wird zumindest ein Datensatz mit einem oder mehreren Werten, Parametern oder Angaben übertragen. Die Übertragung des zumindest einen Datensatzes über die webbasierte Verbindung 6 ist insbesondere bidirektional, was durch den beidseitigen Pfeil 6 symbolisiert ist.

Das Cloud-Computing-Netzwerk 5 weist zumindest ein externes Speicherelement 5A auf, in dem der zumindest eine Datensatz speicherbar ist. Das Cloud-Computing-Netzwerk 5 umfasst insbesondere weitere Hardware- und/ oder Softwareelemente, zum Beispiel einen Mikrocontroller und ein oder mehrere, insbesondere durch den Mikrocontroller ausführbare, Softwareprogramme oder Webapplikationen. Die Webapplikation ist über die Fernbedienungs-Mensch-Maschine-Schnittstelle 4 aufrufbar und/ oder bedienbar.

Die Fernbedienungs-Mensch-Maschine-Schnittstelle kann wahlweise zum Beispiel ein mobiles elektronisches Gerät 4A, insbesondere eine Mobiltelefon, oder ein stationäres elektronisches Gerät 4B, zum Beispiel einen Personalcomputer, aufweisen.

Mit Hilfe der Fernbedienungs-Mensch-Maschine-Schnittstelle 4 wird eine in dem Cloud-Computing-Netzwerk 5, insbesondere in dem externen Speicherelement 5A gespeicherte Webapplikation aufgerufen, um den in dem externen Speicherelement 5A gespeicherten zumindest einen Datensatz aufzurufen und/ oder anzuzeigen und/ oder zu bearbeiten und/ oder zumindest einen Datensatz einzugeben und in dem externen Speicher 5A zu speichern. Um auf die Webapplikation zugreifen zu können, umfasst die Fernbedienungs-Mensch-Maschine-Schnittstelle 4 einen Webbrowser.

Die bidirektionale Kommunikationsverbindung zwischen der Fernbedienungs-Mensch-Maschine-Schnittstelle 4 und dem Cloud-Computing-Netzwerk 5 ist ebenfalls webbasiert und mit dem Bezugszeichen 7 gekennzeichnet.

Die webbasierten Kommunikationsverbindungen 6 und 7 sind drahtlos und/ oder drahtgebunden ausgebildet.

Mit diesem Behandlungs- oder Diagnosesystem 1 ist es möglich, eines oder mehrere der im Vorstehenden beschriebenen Verfahren zur Übertragung von zumindest einem Datensatz zwischen einem dentalen oder dentalchirurgischen Behandlungs- oder Diagnosegerät 2 und einem externen, außerhalb des dentalen oder dentalchirurgischen Behandlungs- oder Diagnosegeräts angeordneten Speicherelement 5A durchzuführen, welche zwecks Vermeidung von Wiederholung nicht noch einmal im Detail beschrieben werden.

Das beschriebene und dargestellte Ausführungsbeispiel dient insbesondere der Veranschaulichung der Erfindung. Die in dem Ausführungsbeispiel offenbarten Merkmale sind daher nicht auf dieses Ausführungsbeispiel beschränkt, sondern sind einzeln oder gemeinsam mit einem oder mehreren Merkmalen eines oder mehrerer anderer beschriebener Ausführungsbeispiele kombinierbar.

## Patentansprüche

1. Verfahren zur Übertragung von zumindest einem Datensatz zwischen einem dentalen oder dentalchirurgischen Behandlungs- oder Diagnosegerät (2) und einem externen, außerhalb des dentalen oder dentalchirurgischen Behandlungs- oder Diagnosegeräts (2) angeordneten Speicherelement (5A), **dadurch gekennzeichnet, dass** der zumindest eine Datensatz webbasiert mittels Internet und eines Cloud-Computing-Netzwerks (5), welches das externe Speicherelement (5A) umfasst, übertragen wird.

2. Verfahren zur Übertragung von zumindest einem Datensatz nach Anspruch 1, **gekennzeichnet durch**
eine bidirektionale Übertragung des zumindest einen Datensatzes zwischen dem dentalen oder dentalchirurgischen Behandlungs- oder Diagnosegerät (2) und dem externen Speicherelement (5A).

3. Verfahren zur Übertragung von zumindest einem Datensatz nach Anspruch 1 oder 2, **dadurch gekennzeichnet, dass**
der zumindest eine Datensatz über eine mit dem Cloud-Computing-Netzwerk (5) kommunikativ verbundene Fernbedienungs-Mensch-Maschine-Schnittstelle (4) angezeigt und/ oder aufgerufen und/ oder bearbeitet wird und/ oder eingegeben und in dem externen Speicher (5A) gespeichert wird.

4. Verfahren zur Übertragung von zumindest einem Datensatz nach Anspruch 3, **dadurch gekennzeichnet, dass**
über die Fernbedienungs-Mensch-Maschine-Schnittstelle (4) eine in dem Cloud-Computing-Netzwerk (5) gespeicherte Webapplikation aufgerufen wird, um den in dem externen Speicherelement (5A) gespeicherten zumindest einen Datensatz aufzurufen und/ oder anzuzeigen und/ oder zu bearbeiten und/ oder einen Datensatz einzugeben und in dem externen Speicher zu speichern.

5. Verfahren zur Übertragung von zumindest einem Datensatz nach einem der vorstehenden Ansprüche, **dadurch gekennzeichnet, dass**
der zumindest eine Datensatz durch einen Mikrocontroller des Cloud-Computing-Netzwerks (5) verarbeitet werden.

6. Verfahren zur Übertragung von zumindest einem Datensatz nach einem der vorstehenden Ansprüche, **dadurch gekennzeichnet, dass**
das externe Speicherelement (5A) Software zur Planung einer Behandlung mit dem dentalen oder dentalchirurgischen Behandlungs- oder Diagnosegerät (2) speichert, wobei der webbasiert mittels Internet und eines Cloud-Computing-Netzwerks (5) übertragene zumindest eine Datensatz einen mit der Software erstellten Behandlungsplan umfasst.

7. Verfahren zur Übertragung von zumindest einem Datensatz nach einem der vorstehenden Ansprüche, **dadurch gekennzeichnet, dass**
der webbasiert mittels Internet und eines Cloud-Computing-Netzwerks (5) übertragene zumindest eine Datensatz Betriebsdaten oder zumindest einen Wert eines Betriebsparameters des dentalen oder dentalchirurgischen Behandlungs- oder Diagnosegeräts (2) und/ oder Behandlungsdaten umfasst.

8. Verfahren zur Übertragung von zumindest einem Datensatz nach Anspruch 7, **dadurch gekennzeichnet, dass**
das externe Speicherelement (5A) Patientendaten speichert, die den übertragenen Betriebsdaten und/ oder Behandlungsdaten zugeordnet werden.

9. Verfahren zur Übertragung von zumindest einem Datensatz nach Anspruch 7 oder 8, **dadurch gekennzeichnet, dass**
die Betriebsdaten zumindest eine Parameter zum Setzen eines dentalen Implantats durch das dentale oder dentalchirurgische Behandlungs- oder Diagnosegerät (2) aufweisen.

10. Verfahren zur Übertragung von zumindest einem Datensatz nach einem der vorstehenden Ansprüche, **dadurch gekennzeichnet, dass**
das dentale oder dentalchirurgische Behandlungs- oder Diagnosegerät (2) ein internes Speicherelement aufweist, das den webbasiert mittels Internet und des Cloud-Computing-Netzwerks (5) übertragenen zumindest einen Datensatz speichert und / oder einen webbasiert mittels Internet und des Cloud-Computing-Netzwerks (5) an das externe Speicherelement (5A) zu übertragenden Datensatz zwischenspeichert.

11. Verfahren zur Übertragung von zumindest einem Datensatz nach einem der vorstehenden Ansprüche, **dadurch gekennzeichnet, dass**
der zumindest eine Datensatz vor der webbasierten Übertragung mittels Internet und eines Cloud-Computing-Netzwerks (5) verschlüsselt und nach der webbasierten Übertragung entschlüsselt wird.

12. Computerprogrammprodukt oder computerlesbares Speichermedium (5A), umfassend Befehle, die bei der Ausführung durch einen Computer diesen veranlassen, das Verfahren nach einem der vorstehenden Ansprüche 1 - 11 auszuführen.

13. Dentales oder dentalchirurgisches Behandlungs- oder Diagnosesystem (1), **gekennzeichnet durch**
ein dentales oder dentalchirurgisches Behandlungs- oder Diagnosegerät (2), ein Cloud-Computing-Netzwerk (5) mit einem externen, außerhalb des dentalen oder dentalchirurgischen Behandlungs- oder Diagnosegeräts (2) angeordneten Speicherelement (5A) und eine Vorrichtung zur Datenverarbeitung mit Mitteln zur Ausführung des Verfahrens nach einem der vorstehenden Ansprüche 1 - 11.

14. Dentales oder dentalchirurgisches Behandlungs- oder Diagnosesystem (1) nach Anspruch 13, **gekennzeichnet durch**
eine Fernbedienungs-Mensch-Maschine-Schnittstelle (4), die kommunikativ mit dem Cloud-Computing-Netzwerk (5) verbunden ist und ausgebildet ist, den webbasiert mittels Internet und des Cloud-Computing-Netzwerks (5) übertragenen zumindest einen Datensatz und/ oder einen im externen Speicherelement (5A) gespeicherten Datensatz anzuzeigen und/ oder aufzurufen und/ oder zu bearbeiten und / oder über die ein Datensatz in den externen Speicher (5A) eingebbar und/ oder speicherbar ist, wobei insbesondere das Cloud-Computing-Netzwerk (5) die Vorrichtung zur Datenverarbeitung umfasst.

15. Dentales oder dentalchirurgisches Behandlungs- oder Diagnosesystem (1) mit einem dentalen oder dentalchirurgischen Behandlungs- oder Diagnosegerät (2) und einer Sende- und Empfangsvorrichtung (3) zur kommunikativen Verbindung mit einer Fernbedienungs-Mensch-Maschine-Schnittstelle (4), **dadurch gekennzeichnet, dass** die Sende- und Empfangsvorrichtung (3) ausgebildet ist, eine webbasierte Anbindung über das Internet und ein Cloud-Computing-Netzwerk (5) an die Fernbedienungs-Mensch-Maschine-Schnittstelle (4) bereitzustellen.

16. Dentales oder dentalchirurgisches Behandlungs- oder Diagnosesystem (1) nach Anspruch 15, **dadurch gekennzeichnet, dass**
die Sende- und Empfangsvorrichtung (3) ein Gateway umfasst.

17. Dentales oder dentalchirurgisches Behandlungs- oder Diagnosesystem (1) nach Anspruch 16, **dadurch gekennzeichnet, dass**
das Gateway (3) ausgebildet ist, einen Datensatz, der über das Gateway (3) übertragen wird, mit einer Datums- und/ oder Zeitangabe zu versehen.

18. Dentales oder dentalchirurgisches Behandlungs- oder Diagnosesystem (1) nach Anspruch 17, **dadurch gekennzeichnet, dass**
der Datensatz, der mit einer Datums- und/ oder Zeitangabe versehen wird, zumindest einen Wert zur Messung der Stabilität eines in einem Knochen fixierten Implantats umfasst.

19. Dentales oder dentalchirurgisches Behandlungs- oder Diagnosesystem (1) nach einem der Ansprüche 15 - 18, **dadurch gekennzeichnet, dass**
in dem Cloud-Computing-Netzwerk (5) zumindest eine Webapplikation gespeichert ist, die über die Fernbedienungs-Mensch-Maschine-Schnittstelle (4) aufrufbar ist.

## Geänderte Patentansprüche

### Geänderte Patentansprüche gemäss Regel 137(2) EPÜ.

1. Verfahren zur Übertragung von zumindest einem Datensatz zwischen einem dentalen oder dentalchirurgischen Behandlungs- oder Diagnosegerät (2) und einem externen, außerhalb des dentalen oder dentalchirurgischen Behandlungs- oder Diagnosegeräts (2) angeordneten Speicherelement (5A), wobei der zumindest eine Datensatz webbasiert mittels Internet und eines Cloud-Computing-Netzwerks (5), welches das externe Speicherelement (5A) umfasst, übertragen wird, **dadurch gekennzeichnet, dass**
das externe Speicherelement (5A) Software zur Planung einer Behandlung mit dem dentalen oder dentalchirurgischen Behandlungs- oder Diagnosegerät (2) speichert, wobei der webbasiert mittels Internet und eines Cloud-Computing-Netzwerks (5) übertragene zumindest eine Datensatz einen mit der Software erstellten Behandlungsplan zur Implantation eines dentalen Implantats umfasst, und wobei der zumindest eine, den Behandlungsplan enthaltende Datensatz über eine mit dem Cloud-Computing-Netzwerk (5) kommunikativ verbundene Fernbedienungs-Mensch-Maschine-Schnittstelle (4) durch einen Anwender bearbeitet oder eingegeben und in dem externen Speicher (5A) gespeichert wird.

2. Verfahren zur Übertragung von zumindest einem Datensatz nach Anspruch 1, **gekennzeichnet durch**
eine bidirektionale Übertragung des zumindest einen Datensatzes zwischen dem dentalen oder dentalchirurgischen Behandlungs- oder Diagnosegerät (2) und dem externen Speicherelement (5A).

3. Verfahren zur Übertragung von zumindest einem Datensatz nach Anspruch 1 oder 2, **dadurch gekennzeichnet, dass**
der zumindest eine Datensatz Angaben zu einem zu implantierenden oder implantierten Implantat aufweist.

4. Verfahren zur Übertragung von zumindest einem Datensatz nach einem der vorstehenden Ansprüche, **dadurch gekennzeichnet, dass**
über die Fernbedienungs-Mensch-Maschine-Schnittstelle (4) eine in dem Cloud-Computing-Netzwerk (5) gespeicherte Webapplikation aufgerufen wird, um den in dem externen Speicherelement (5A) gespeicherten zumindest einen Datensatz aufzurufen und/ oder anzuzeigen und/ oder zu bearbeiten und/ oder einen Datensatz einzugeben und in dem externen Speicher zu speichern.

5. Verfahren zur Übertragung von zumindest einem Datensatz nach einem der vorstehenden Ansprüche, **dadurch gekennzeichnet, dass**
der zumindest eine Datensatz durch einen Mikrocontroller des Cloud-Computing-Netzwerks (5) verarbeitet wird.

6. Verfahren zur Übertragung von zumindest einem Datensatz nach einem der vorstehenden Ansprüche, **dadurch gekennzeichnet, dass**
das externe Speicherelement (5A) eine Implantatbibliothek mit Daten oder Werten zu mehreren Implantaten speichert.

7. Verfahren zur Übertragung von zumindest einem Datensatz nach Anspruch 6, **dadurch gekennzeichnet, dass**
über eine Webapplikation und die Fernbedienungs-Mensch-Maschine-Schnittstelle (4) ein Implantat, das in einer dentalchirurgischen Implantation implantiert wird, mit einem dem Implantat zugeordneter Datensatz und/ oder Behandlungswert aus der Implantatbibliothek ausgewählt und in den Behandlungsplan übernommen wird.

8. Verfahren zur Übertragung von zumindest einem Datensatz nach einem der vorstehenden Ansprüche, **dadurch gekennzeichnet, dass**
der webbasiert mittels Internet und eines Cloud-Computing-Netzwerks (5) übertragene zumindest eine Datensatz Betriebsdaten oder zumindest einen Wert eines Betriebsparameters des dentalen oder dentalchirurgischen Behandlungs- oder Diagnosegeräts (2) und/ oder Behandlungsdaten umfasst.

9. Verfahren zur Übertragung von zumindest einem Datensatz nach Anspruch 8, **dadurch gekennzeichnet, dass**
das externe Speicherelement (5A) Patientendaten speichert, die den übertragenen Betriebsdaten und/ oder Behandlungsdaten zugeordnet werden.

10. Verfahren zur Übertragung von zumindest einem Datensatz nach einem der vorstehenden Ansprüche, **dadurch gekennzeichnet, dass**
mit einer Stellvorrichtung (2B) des dentalen oder dentalchirurgische Behandlungs- oder Diagnosegeräts (2) zumindest einen Wert des webbasiert mittels Internet und des Cloud-Computing-Netzwerks übertragenen zumindest einen Datensatzes, verändert wird.

11. Verfahren zur Übertragung von zumindest einem Datensatz nach einem der vorstehenden Ansprüche, **dadurch gekennzeichnet, dass**
das dentale oder dentalchirurgische Behandlungs- oder Diagnosegerät (2) ein internes Speicherelement aufweist, das den webbasiert mittels Internet und des Cloud-Computing-Netzwerks (5) übertragenen zumindest einen Datensatz speichert und / oder einen webbasiert mittels Internet und des Cloud-Computing-Netzwerks (5) an das externe Speicherelement (5A) zu übertragenden Datensatz zwischenspeichert.

12. Verfahren zur Übertragung von zumindest einem Datensatz nach einem der vorstehenden Ansprüche, **dadurch gekennzeichnet, dass**
der zumindest eine Datensatz vor der webbasierten Übertragung mittels Internet und eines Cloud-Computing-Netzwerks (5) verschlüsselt und nach der webbasierten Übertragung entschlüsselt wird.

13. Verfahren zur Erstellung eines Behandlungsplans für eine dentalchirurgische Implantation, bei dem der Behandlungsplan zwischen einem dentalchirurgischen Behandlungsgerät (2) und einem externen, außerhalb des dentalchirurgischen Behandlungsgeräts (2) angeordneten Speicherelement (5A) webbasiert mittels Internet und eines Cloud-Computing-Netzwerks (5), welches das externe Speicherelement (5A) umfasst, übertragen wird, umfassend: (A) zur Verfügung stellen einer Fernbedienungs-Mensch-Maschine-Schnittstelle (4), die kommunikativ mit dem Cloud-Computing-Netzwerk (5) verbunden ist oder wird; (B) Aufrufen einer in dem externe Speicherelement (5A) des Cloud-Computing-Netzwerks (5) gespeicherten Webapplikation zur Erstellung eines Behandlungsplans einer dentalchirurgischen Implantation mit der Fernbedienungs-Mensch-Maschine-Schnittstelle (4); (C) Eingabe und/ oder Auswahl und/ oder Änderung zumindest eines Datensatzes und/ oder Parameterwertes für die dentalchirurgischen Implantation über die aufgerufene Webapplikation und Übernahme oder Speicherung dieses Datensatzes und/ oder Parameterwertes in den Behandlungsplan; und (D) webbasiertes Übertragen des Behandlungsplans einschließlich des zumindest einen Datensatzes und/ oder Parameterwertes mittels Internet von dem Cloud-Computing-Netzwerk (5) auf das dentalchirurgische Behandlungsgerät (2).

14. Computerprogrammprodukt oder computerlesbares Speichermedium (5A), umfassend Befehle, die bei der Ausführung durch einen Computer diesen veranlassen, das Verfahren nach einem der vorstehenden Ansprüche 1 - 13 auszuführen.

15. Dentales oder dentalchirurgisches Behandlungs- oder Diagnosesystem (1), **gekennzeichnet durch**
ein dentales oder dentalchirurgisches Behandlungs- oder Diagnosegerät (2), ein Cloud-Computing-Netzwerk (5) mit einem externen, außerhalb des dentalen oder dentalchirurgischen Behandlungs- oder Diagnosegeräts (2) angeordneten Speicherelement (5A), eine Fernbedienungs-Mensch-Maschine-Schnittstelle (4), die kommunikativ mit dem Cloud-Computing-Netzwerk (5) verbunden und ausgebildet ist, den webbasiert mittels Internet und des Cloud-Computing-Netzwerks (5) übertragenen zumindest einen Datensatz und/ oder einen im externen Speicherelement (5A) gespeicherten Datensatz durch einen Anwender zu bearbeiten oder in den externen Speicher (5A) einzugeben und zu speichern, und eine Vorrichtung zur Datenverarbeitung mit Mitteln zur Ausführung des Verfahrens nach einem der vorstehenden Ansprüche 1 - 13.
